(19) Europäisches Patentamt
European Patent Office
Office européen des brevets

(11) **EP 4 115 952 A1**

(12) **EUROPEAN PATENT APPLICATION**
published in accordance with Art. 153(4) EPC

(43) Date of publication:
**11.01.2023 Bulletin 2023/02**

(21) Application number: **21765319.5**

(22) Date of filing: **26.02.2021**

(51) International Patent Classification (IPC):
*A61Q 17/04* (2006.01)    *A61K 8/06* (2006.01)
*A61K 8/27* (2006.01)    *A61K 8/29* (2006.01)
*A61K 8/55* (2006.01)    *A61K 8/81* (2006.01)

(52) Cooperative Patent Classification (CPC):
**A61K 8/06; A61K 8/27; A61K 8/29; A61K 8/55;**
**A61K 8/81; A61Q 17/04**

(86) International application number:
**PCT/JP2021/007532**

(87) International publication number:
**WO 2021/177188 (10.09.2021 Gazette 2021/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.03.2020  JP 2020035165**

(71) Applicant: **Kao Corporation
Chuo-ku
Tokyo 103-8210 (JP)**

(72) Inventor: **INOUE, Yoshihiro
Odawara-shi, Kanagawa 250-0002 (JP)**

(74) Representative: **Hoffmann Eitle
Patent- und Rechtsanwälte PartmbB
Arabellastraße 30
81925 München (DE)**

(54) **OIL-IN-WATER-TYPE EMULSIFIED COSMETIC MATERIAL**

(57)    An oil-in-water emulsion cosmetic material containing the following component (A) to (C): Component (A): a hydrophobized fine-particulate metal oxide having an average primary particle diameter of less than 100 nm, Component (B): an aqueous phase thickener containing a structure derived from 2-acrylamido-2-methylpropanesulfonic acid, Component (C): an amphoteric surfactant, wherein the content of the component (A) is 7% by mass or more and 30% by mass or less.

**Description**

Field of the Invention

The present invention relates to an oil-in-water emulsion cosmetic material having an excellent UV protective effect.

Background of the Invention

In recent years, as the importance of protection against ultraviolet rays in daily life has increased, the need for sunscreen cosmetics has increased. Under such market needs, oil-in-water emulsion-type sunscreen cosmetics which feel refreshing in use and are easy to use continuously have been developed.

In these oil-in-water emulsion cosmetics, a UV absorbent or a UV scattering agent is used for increasing the UV protective effect, and especially as the UV scattering agent, a metal oxide powder of zinc oxide or titanium oxide is used.

However, when a metal oxide powder is blended in an oil-in-water emulsion cosmetic material, there occur some problems that the feel in use worsens and the stability of the metal oxide powder in the oil-in-water emulsion cosmetic material is not sufficient.

For solving such problems, for example, JP2018-108994A (PTL 1) discloses, for the purpose of providing an oil-in-water emulsion composition capable of stably containing a hydrophobized powder, excellent in emulsion system stability in storage at high temperatures or in reception of shear force, and excellent in feeling in use such as freshness, an oil-in-water emulsion composition containing a polyoxyethylene alky ether or a polyoxyethylene alkenyl ether such that the carbon number of the alkyl group or the alkenyl group therein falls within a predetermined range and such that the average addition molar number of ethylene oxide therein falls within a predetermined range, and a phospholipid, a hydrophobized powder, and water, in which the content mass ratio of the polyoxyethylene alkyl ether or the polyoxyethylene alkenyl ether to the phospholipid falls within a predetermined range.

JP2019-94280A (PTL 2) discloses, for the purpose of providing an oil-in-water emulsion cosmetic material having an excellent UV protective effect, having good time stability, etc. and having excellent rub resistance, an oil-in-water emulsion cosmetic material which contains an oil-soluble organic UV absorbentcontaining liquid oil, a salt-resistance water-soluble polymer, and a hydrophobized fine-particulate metal oxide powder, and in which the liquid oil contains a component having an IOB value of 0.10 or more in an amount of 40% by mass or more, the liquid oil content is 3% by mass or more and 30% by mass or less relative to the total amount of the cosmetic material, and the content mass ratio of the hydrophobized fine-particulate metal oxide powder to the liquid oil is 0.4 or more and 1.0 or less.

Summary of the Invention

The present invention relates to an oil-in-water emulsion cosmetic material containing the following component (A), component (B) and component (C):

Component (A): a hydrophobized fine-particulate metal oxide having an average primary particle diameter of less than 100 nm,
Component (B): an aqueous phase thickener containing a structure derived from 2-acrylamido-2-methylpropanesulfonic acid,
Component (C): an amphoteric surfactant, wherein:
the content of the component (A) is 7% by mass or more and 30% by mass or less.

Detailed Description of the Invention

In blending a large amount of a metal oxide powder in an oil-in-water emulsion cosmetic material for enhancing the UV protective effect, emulsion stability is as yet not sufficient, and the preparation is less likely to disintegrate at the time of application, and may give a friction feeling. Further, after application, a tensive feeling may be given.

The present invention relates to an oil-in-water emulsion cosmetic material having an excellent UV protective effect, excellent in emulsion stability, readily disintegrable in application as a preparation, and excellent in the feeling in use in that a friction feeling at the time of application and a tensive feeling after application are suppressed.

The present inventor has found that, using a hydrophobized fine-particulate metal oxide having the average primary particle diameter thereof falls within a predetermined range, an aqueous phase thickener having a specific structure and an amphoteric surfactant and controlling the content of the hydrophobized fine-particulate metal oxide to fall within a predetermined range, an oil-in-water emulsion cosmetic can be obtained, which is excellent in UV protection performance and has high emulsion stability and, in addition, which is readily disintegrable in application as a preparation, and is excellent in the feeling in use in that a friction feeling at the time of application and a tensive feeling after application are suppressed.

Specifically, the present invention relates to an oil-in-water emulsion cosmetic material containing the following component (A), component (B) and component (C):

Component (A): a hydrophobized fine-particulate metal oxide having an average primary particle diameter of less than 100 nm,
Component (B): an aqueous phase thickener containing a structure derived from 2-acrylamido-2-methylpropanesulfonic acid,
Component (C): an amphoteric surfactant, wherein:
the content of the component (A) is 7% by mass or more and 30% by mass or less.

The present invention can provide an oil-in-water emulsion cosmetic material having an excellent UV protective effect, excellent in emulsion stability, readily disintegrable in application as a preparation, and excellent in the feeling in use in that a friction feeling at the time of application and a tensive feeling after application are suppressed.

[Oil-in-water emulsion cosmetic material]

The oil-in-water emulsion cosmetic material of the present invention (hereinafter also referred to as "emulsion cosmetic material") is an oil-in-water emulsion cosmetic material containing the following component (A), component (B) and component (C):

Component (A): a hydrophobized fine-particulate metal oxide having an average primary particle diameter of less than 100 nm,
Component (B): an aqueous phase thickener containing a structure derived from 2-acrylamido-2-methylpropanesulfonic acid,
Component (C): an amphoteric surfactant, wherein:
the content of the component (A) is 7% by mass or more and 30% by mass or less.

In the present invention, "hydrophobized fine-particulate metal oxide" is a fine-particulate metal oxide whose surface has been hydrophobized.

The oil-in-water emulsion cosmetic material of the present invention has an excellent UV protective effect, and is excellent in emulsion stability and readily disintegrable in application as a preparation, and can give an excellent feeling in use in that a friction feeling at the time of application and a tensive feeling after application are suppressed. Though not clear, the reason is considered as follows.

The fine-particulate metal oxide contained in the oil-in-water emulsion cosmetic material of the present invention has an average primary particle diameter of less than 100 nm and the surface thereof has been hydrophobized. With that, the oil-in-water emulsion cosmetic material further contains an aqueous phase thickener that contains a structure derived from 2-acrylamido-2-methylpropanesulfonic acid as a thickener to thicken an aqueous phase of a continuous phase, and an amphoteric surfactant, and therefore it is considered that, even when the content of the fine-particulate metal oxide is high, in application of the fine-particulate metal oxide to the oil-in-water emulsion cosmetic material, the fine-particulate metal oxide can be made to exist stably in an oily phase. As a result, it is considered that emulsion stability can improve, and UV rays can be efficiently scattered to enhance UV protection performance, and further, at the time of application, the cosmetic material can readily disintegrate as a preparation, and can suppress a friction feeling at the time of application and a tensive feeling after application.

<Component (A): hydrophobized fine-particulate metal oxide having an average primary particle diameter of less than 100 nm>

The oil-in-water emulsion cosmetic material of the present invention contains a hydrophobized fine-particulate metal oxide having an average primary particle diameter of less than 100 nm as a component (A).

The metal oxide of the component (A) is, from the viewpoint of scattering UV rays and enhancing the UV protective effect, preferably at least one selected from the group consisting of zinc oxide, titanium oxide and cerium oxide, more preferably at least one selected from the group consisting of zinc oxide and titanium oxide. The fine-particulate metal oxide can contain a divalent or more polyvalent metal, and a metal such as iron, zirconium, calcium, manganese, magnesium and yttrium or an oxide thereof can be contained in the metal oxide either singly or as a combination of two or more kinds thereof.

The average primary particle diameter of the component (A) is, from the viewpoint of enhancing the UV protective effect and the viewpoint of improving emulsion stability, less than 100 nm, preferably 90 nm or less, more preferably 70 nm or less, even more preferably 50 nm or less, further more preferably 40 nm or less, further more preferably 30 nm or less, and is preferably 1 nm or more, more preferably 5 nm or more, even more preferably 10 nm or more. More specifically, the average primary particle diameter of the component (A) is, from the viewpoint of enhancing the UV protective effect and the viewpoint of improving emulsion stability, preferably 1 to 90 nm, more preferably 1 to 70 nm, even more preferably 1 to 50 nm, further more preferably 5 to 50 nm, further more preferably 10 to 50 nm, further more preferably 10 to 40 nm, further more preferably 10 to 30 nm.

Examples of the shape of the component (A) include spherical, flaky, rodlike, spindle-shaped, acicular and amorphous states, but any arbitrary shape can be used here so far as the average primary particle diameter thereof falls within the above-mentioned range.

The average primary particle diameter of the component (A) in the present invention can be determined on an image observed with a transmission electron microscope (TEM). Specifically, observation is performed with a TEM at an observation magnification of 50,000 times, and the maximum minor diameter of 300 primary particles in the observed image is measured to calculate a number average value thereof. Here, the maximum minor diameter means a minor diameter having a maximum length of the minor diameter that crosses a major diameter at right angles in the case where the component (A) has a shape except a flaky shape. In the case where the component (A) has a flaky shape, the thickness of 300 primary particles in the image observed under the same condition as above is measured to calculate a number average value thereof. Specifically, the average primary particle diameter is measured according to the method described in the section of Examples.

Hydrophobization for the component (A) includes silicone treatment; alkylalkoxysilane treatment; fatty acid treatment; fluorine-containing compound treatment with a perfluoroalkylphosphate ester or a perfluoroalcohol; amino acid treatment with an N-acylglutamic acid; lecithin treatment; metal soap treatment; alkyl phosphate ester treatment; and ASI treatment with an N-acylamino acid metal salt (sodium lauroyl aspartate) and zinc chloride and an alkoxy titanium alkylate (isopropyl titanium triisostearate).

One alone or two or more kinds of these surface treatments may be carried out either singly or as combined.

Among these, the hydrophobization for the component (A) is, from the viewpoint of increasing the content of the fine-particulate metal oxide in the oil-in-water emulsion cosmetic material to enhance the UV protective effect and the viewpoint of improving emulsion stability and disintegrability of preparations at the time of application, and from the viewpoint of suppressing the friction feeling at the time of application and the tensive feeling after application, preferably at least one selected from the group consisting of silicone treatment, alkylalkoxysilane treatment and fatty acid treatment.

Examples of the surface treatment agent for use in silicone treatment include various silicone oils such as methylpolysiloxane, dimethylpolysiloxane, methylphenylpolysiloxane, methyl hydrogen polysiloxane, methylcyclopolysiloxane, dodecamethylcyclohexasiloxane, tetradecamethylhexasiloxane, dimethylsiloxane/methyl(polyoxyethylene)-siloxane/methyl(polyoxypropylene)siloxane copolymer, dimethylsiloxane/methyl(polyoxyethylene)siloxane copolymer, dimethylsiloxane/methyl(polyoxypropylene)siloxane copolymer, dimethylsiloxane/methylcetyloxysiloxane copolymer, dimethylsiloxane/methylstearoxysiloxane copolymer, and (alkyl acrylate/dimethicone) copolymer. Among these, from the viewpoint of increasing the dispersibility of the component (A) in the emulsion cosmetic material and improving the UV protective effect, emulsion stability and disintegrability of preparations at the time of application, and from the viewpoint of suppressing the friction feeling at the time of application and the tensive feeling after application, preferred are methyl hydrogen polysiloxane and dimethylpolysiloxane.

As the surface treatment agent for use in alkylalkoxysilane treatment, from the viewpoint of increasing the dispersibility of the component (A) in the emulsion cosmetic material and improving the UV protective effect, emulsion stability and disintegrability of preparations at the time of application, and from the viewpoint of suppressing the friction feeling at the time of application and the tensive feeling after application, preferred is one having a linear or branched alkyl group with 6 or more and 20 or less carbon atoms, and more preferred are octyltriethoxysilane and octyltrimethoxysilane.

The surface treatment agent for use in fatty acid treatment includes a linear or branched-chain higher fatty acid having 12 or more and 22 or less carbon atoms. Above all, from the viewpoint of improving the UV protective effect and the viewpoint of improving emulsion stability and disintegrability of preparations at the time of application, and from the viewpoint of suppressing the friction feeling at the time of application and the tensive feeling after application, preferred is a linear or branched-chain higher fatty acid having 14 or more and 22 or less carbon atoms, more preferred is a linear or branched-chain higher fatty acid having 16 or more and 20 or less carbon atoms, and even more preferred are stearic acid and isostearic acid.

One alone or two or more kinds of the above-mentioned surface treatment agents can be used either singly or as combined.

Commercial products of the hydrophobized fine-particulate zinc oxide include FINEX series (by Sakai Chemical Industry Co., Ltd.), MZ series and MZY series (all by Tayca Corporation).

Commercial products of the hydrophobized fine-particulate titanium oxide include STR series (by Sakai Chemical Industry Co., Ltd.), TTO-55 series and TTO-51 series (all by Ishihara Sangyo Kaisha, Ltd.), MT series and MTY series (all by Tayca Corporation).

The hydrophobization throughput in the component (A) is, from the viewpoint of increasing the dispersibility of the component (A) in the emulsion cosmetic material and improving the UV protective effect, emulsion stability and disintegrability of preparations at the time of application, and from the viewpoint of suppressing the friction feeling at the time of application and the tensive feeling after application, preferably 0.1% by mass or more relative to 100% by mass of the component (A), and is preferably 40% by mass or less, more preferably 30% by mass or less.

In the present invention, the mass and the average primary particle diameter of the component (A) mean the mass and the average primary particle diameter thereof including the surface treatment agent.

The content of the component (A) in the emulsion cosmetic material of the present invention is, from the viewpoint of increasing the UV protective effect, 7% by mass or more, preferably 9% by mass or more, more preferably 10% by mass or more, even more preferably 13% by mass or more, and is, from the viewpoint of improving emulsion stability and disintegrability of preparation at the time of application, and also from the viewpoint of suppressing the friction feeling at the time of application and the tensive feeling after application, 30% by mass or less, preferably 27% by mass or less, more preferably 25% by mass or less, even more preferably 23% by mass or less, further more preferably 20% by mass or less. More specifically, from the viewpoint of increasing the UV protective effect, from the viewpoint of improving emulsion stability and disintegrability of preparation at the time of application, and from the viewpoint of suppressing the friction feeling at the time of application and the tensive feeling after application, the content is preferably 9 to 27% by mass, more preferably 10 to 25% by mass, even more preferably 10 to 23% by mass, further more preferably 13 to 20% by mass.

The component (A) preferably contains a hydrophobized fine-particulate zinc oxide from the viewpoint of transparency in application to skin. In the case where the component (A) contains a hydrophobized fine-particulate zinc oxide, the content of the hydrophobized fine-particulate zinc oxide in the component (A) is, from the same viewpoint as above, preferably 60% by mass or more, more preferably 70% by mass or more, even more preferably 80% by mass or more, further more preferably 90% by mass or more.

The component (A) preferably contains a hydrophobized fine-particulate titanium oxide from the viewpoint of the UV protective effect. In the case where the component (A) contains a hydrophobized fine-particulate titanium oxide, the content of the hydrophobized fine-particulate titanium oxide in the component (A) is, from the same viewpoint as above, preferably 50% by mass or more, more preferably 55% by mass or more, even more preferably 60% by mass or more, further more preferably 65% by mass or more.

<Component (B): Aqueous phase thickener containing structure derived from 2-acrylamido-2-methylpropanesulfonic acid>

The oil-in-water emulsion cosmetic material of the present invention contains an aqueous phase thickener containing a structure derived from 2-acrylamido-2-methylpropanesulfonic acid (acryloyldimethyltauric acid) (hereinafter also referred to as "acryloyldimethyltauric acid" or "AMPS"), as a component (B).

Not specifically limited, the component (B) may be any one that contains a structure derived from 2-acrylamido-2-methylpropanesulfonic acid (acryloyldimethyltauric acid), and has an effect of thickening an aqueous phase of a continuous phase, but is, from the viewpoint of increasing the UV protective effect, the viewpoint of improving emulsion stability and disintegrability of preparations at the time of application, and from the viewpoint of suppressing the friction feeling at the time of application and the tensive feeling after application, preferably a water-soluble polymer containing a structural unit derived from acryloyldimethyltauric acid.

The water-soluble polymer containing a structural unit derived from acryloyldimethyltauric acid includes a homopolymer of acryloyldimethyltauric acid, and a copolymer containing a structural unit derived from acryloyldimethyltauric acid, and is preferably a copolymer containing a structural unit derived from acryloyldimethyltauric acid (AMPS).

Examples of the copolymer containing a structural unit derived from AMPS include (AMPS/acrylic acid) copolymer, (AMPS/acrylamide) copolymer, (AMPS/acrylic acid/acrylamide) copolymer, (AMPS/dimethylacrylamide) copolymer, (AMPS/hydroxyethyl acrylate) copolymer, (AMPS/vinylpyrrolidone) copolymer, (AMPS/vinylformamide) copolymer, (AMPS/polyoxyethylene alkyl methacrylate (average addition molar number of ethylene oxide: 4 or more and 30 or less, preferably 10 or more and 30 or less)) copolymer, and salts thereof. More specifically, there are mentioned (Na acryloyldimethyltaurate/hydroxyethyl acrylate) copolymer (INCI name: Hydroxyethyl Acrylate/Sodium Acryloyldimethyltaurate Copolymer), (Na acryloyldimethyltaurate/Na acrylate) copolymer (INCI name: Sodium Acrylate/Sodium Acryloyldimethyltaurate Copolymer), (acryloyldimethyltaurate/Na acrylate/dimethylacrylamide) crosspolymer (INCI name: Sodium Acrylate/Acryloyldimethyltaurate/Dimethylacrylamide Crosspolymer), (ammonium acryloyldimethyltaurate/vinyl pyrrolidone) copolymer (INCI name :Ammonium Acryloyldimethyltaurate/VP Copolymer), (Na acryloyldimethyltaurate/acrylic acid/acrylamide/Na acrylate) copolymer (INCI name: Polyacrylate-13), (ammonium acryloyldimethyltaurate/dimethylacrylamide/lauryl methacrylate/laureth-4 methacrylate) crosspolymer (INCI name: Polyacrylate Crosspolymer-6), (ammonium acryloyldimethyltaurate/beheneth-25 methacrylate) crosspolymer (INCI name: Ammonium Acryloyldimethyltaurate/Beheneth-25 Methacrylate Crosspolymer), and (ammonium acryloyldimethyltaurate/steareth-25 methacrylate) crosspolymer (INCI name: Ammonium Acryloyldimethyltaurate/Steareth-25 Methacrylate Crosspolymer).

Among these, the AMPS-derived structural unit-containing copolymer is, from the viewpoint of increasing the UV protective effect, from the viewpoint of improving emulsion stability and disintegrability of preparations at the time of application, and from the viewpoint of suppressing the friction feeling at the time of application and the tensive feeling after application, preferably a copolymer further containing, in addition to the AMPS-derived structural unit, a structural unit derived from an acrylic monomer as another monomer except AMPS, such as acrylic acid, acrylamide, dimethylacrylamide, alkyl acrylate, alkyl methacrylate, hydroxyalkyl acrylate and polyoxyethylene alkyl methacrylate (average addition molar number of ethylene oxide: 4 or more and 30 or less, preferably 10 or more and 30 or less). One alone or two or more kinds of the acrylic monomers may be used either singly or as combined.

Commercial products of the component (B) include "SIMULGEL EG"((Na acryloyldimethyltaurate/Na acrylate)copolymer, isohexadecane, Polysorbate 80), "SEPIMAX ZEN" ((ammonium acryloyldimethyltaurate/dimethylacrylamide/lauryl methacrylate/laureth-4 methacrylate) crosspolymer), "SEPINOV EMT 10" ((Na acryloyldimethyltaurate/hydroxyethyl acrylate) copolymer), "SIMULGEL NS" ((Na acryloyldimethyltaurate/hydroxyethyl acrylate) copolymer, squalane, Polysorbate 60, water), "SIMULGEL FL" ((Na acryloyldimethyltaurate/hydroxyethyl acrylate) copolymer, isohexadecane, Polysorbate 60, water), "SEPIPLUS S" ((Na acryloyldimethyltaurate/hydroxyethyl acrylate) copolymer, polyisobutene, PEG-7 trimethylolpropane palm oil alkyl ether, water), and "SEPIPLUS 400" ((Na acryloyldimethyltaurate/acrylic acid/acrylamide/Na acrylate) copolymer, polyisobutene, Polysorbate 20, water) (all by SEPPIC Corporation); and "Aristoflex AVC" ((ammonium acryloyldimethyltaurate/vinylpyrrolidone) copolymer), "Aristoflex HMB" ((ammonium acryloyldimethyltaurate/beheneth-25 methacrylate) crosspolymer), and "Aristoflex HMS" ((ammonium acryloyldimethyltaurate/steareth-25 methacrylate)crosspolymer) (all by Clariant Corporation).

Among these, the component (B) is preferably a copolymer containing an AMPS-derived structural unit, more preferably a copolymer containing an AMPS-derived structural unit and an acrylic monomer-derived structural unit, even more preferably at least one selected from the group consisting of (Na acryloyldimethyltaurate/Na acrylate) copolymer, (ammonium acryloyldimethyltaurate/beheneth-25 methacrylate) crosspolymer, and (ammonium acryloyldimethyltaurate/dimethylacrylamide/lauryl methacrylate/laureth-4 methacrylate) crosspolymer, further more preferably (Na acryloyldimethyltaurate/Na acrylate) copolymer.

The content of the component (B) in the emulsion cosmetic material of the present invention is, from the viewpoint of increasing the UV protective effect and from the viewpoint of improving emulsion stability, preferably 0.1% by mass or more, more preferably 0.15% by mass or more, even more preferably 0.2% by mass or more, further more preferably 0.25% by mass or more, and is, from the viewpoint of improving disintegrability of preparations at the time of application and from the viewpoint of suppressing the friction feeling at the time of application, preferably 3% by mass or less, more preferably 2% by mass or less, even more preferably 1% by mass or less, further more preferably 0.5% by mass or less. More specifically, the content is preferably 0.1 to 3% by mass, more preferably 0.15 to 2% by mass, even more preferably 0.2 to 1% by mass, further more preferably 0.25 to 0.5% by mass.

The content ratio by mass of the component (A) to the component (B) in the emulsion cosmetic material of the present invention [(A)/(B)] is, from the viewpoint of improving disintegrability of preparations at the time of application and from the viewpoint of suppressing the friction feeling at the time of application, preferably 10 or more, more preferably 15 or more, even more preferably 20 or more, further more preferably 30 or more, and is, from the viewpoint of increasing the UV protective effect and form the viewpoint of improving emulsion stability, preferably 90 or less, more preferably 80 or less, even more preferably 70 or less, further more preferably 50 or less. More specifically, the content ratio is preferably 10 to 90, more preferably 15 to 80, even more preferably 20 to 70, further more preferably 30 to 50.

<Component (C): Amphoteric surfactant>

The oil-in-water emulsion cosmetic material of the present invention contains an amphoteric surfactant as a component (C).

The amphoteric surfactant includes a betaine-type surfactant, an amine oxide-type surfactant, and an amino acid-type surfactant. Among these, from the viewpoint of increasing the UV protective effect and the viewpoint of improving emulsion stability and disintegrability of preparations at the time of application, and also from the viewpoint of suppressing the friction feeling at the time of application and the tensive feeling after application, preferred is a betaine-type surfactant.

Examples of the amphoteric surfactant include carboxybetaine types such as betaine alkyldimethylaminoacetate, and fatty acid amide propylbetaine; sulfobetaine types such as alkylsulfobetaine, and alkylhydroxysulfobetaine; imidazole-based betaine types such as alkylcarboxymethylhydroxyethylimidazolinium betaine; and phosphorbetaine types such as phosphatidylcholine, phosphatidylethanolamine, phosphatidylcerine and laurylhydroxyphosphobetaine.

Among these, from the viewpoint of increasing the UV protective effect and the viewpoint of improving emulsion stability and disintegrability of preparations at the time of application, and also from the viewpoint of suppressing the friction feeling at the time of application and the tensive feeling after application, preferred are phosphobetaine-type surfactants, more preferred are phospholipid-containing lecithins such as phosphatidylcholine and phosphatidylethanolamine, or hydrogenated lecithins that are hydrogenation products of the lecithins, and even more preferred are hydrogenated lecithins.

Hydrogenated lecithins are hydrogenation products of lecithins that are natural substances extracted or purified from animals or plants, or chemical synthetic substances. The natural substances are, from the viewpoint of reducing the load to skin and the viewpoint of availability, preferably hydrogenated soybean lecithin and hydrogenated egg yolk lecithin that are extracted products or purified products from soybean or egg yolk, more preferably hydrogenated soybean lecithin.

The hydrogenated lecithin as the component (C) is, from the viewpoint of increasing the UV protective effect and the viewpoint of improving emulsion stability and disintegrability of preparations at the time of application, and also from the viewpoint of suppressing the friction feeling at the time of application and the tensive feeling after application, preferably one containing phosphatidylcholine.

The content of phosphatidylcholine in the hydrogenated lecithin is preferably 50% by mass or more, more preferably 55% by mass or more, even more preferably 60% by mass or more, further more preferably 65% by mass or more, and is preferably 95% by mass or less, more preferably 90% by mass or less, even more preferably 85% by mass or less, further more preferably 80% by mass or less. More specifically, the content is preferably 50 to 95% by mass, more preferably 55 to 90% by mass, even more preferably 60 to 85% by mass, further more preferably 65 to 80% by mass.

The content of phosphatidylcholine in the hydrogenated lecithin can be determined by analysis according to a method of thin-layer chromatography (TLC) or high-performance liquid chromatography (HPLC), or a method using Iatroscan (by Iatron, Inc.). For example, one method is described in JP2001-186898A, in which an organic solvent containing a hydrogenated lecithin is spotted by TLC, developed with chloroform/methanol/acetate = 65/25/10, sprayed with 50 mass% sulfuric acid/ethanol, then heated, and the hydrogenated lecithin is analyzed with a densitometer.

Commercial products of the component (C) include "PhosphoLipid PCSH70" (hydrogenated soybean lecithin (phosphatidylcholine content: about 70% by mass); by Nippon Fine Chemical Co., Ltd.), "Coatsome NC-21" (hydrogenated soybean lecithin; by NOF Corporation), "Lecinol S-10E" and "Lecinol S-10EX" (hydrogenated soybean lecithin; by Nikko Chemicals Co., Ltd.).

The content of the component (C) in the emulsion cosmetic material of the present invention is, from the viewpoint of increasing the UV protective effect and the viewpoint of improving emulsion stability, preferably 0.1% by mass or more, more preferably 0.2% by mass or more, even more preferably 0.3% by mass or more, and is, from the viewpoint of improving disintegrability of preparations at the time of application, and from the viewpoint of suppressing the friction feeling at the time of application and the tensive feeling after application, preferably 3% by mass or less, more preferably 2% by mass or less, even more preferably 1% by mass or less, further more preferably 0.7% by mass or less. More specifically the content is preferably 0.1 to 3% by mass, more preferably 0.2 to 2% by mass, even more preferably 0.3 to 1% by mass, further more preferably 0.3 to 0.7% by mass.

The content ratio by mass of the component (A) to the component (C) in the emulsion cosmetic material of the present invention [(A)/(C)] is, from the viewpoint of improving disintegrability of preparations at the time of application, and from the viewpoint of suppressing the friction feeling at the time of application and the tensive feeling after application, preferably 5 or more, more preferably 7.5 or more, even more preferably 10 or more, further more preferably 20 or more, and is, from the viewpoint of increasing the UV protective effect and the viewpoint of improving emulsion stability, preferably 80 or less, more preferably 75 or less, even more preferably 70 or less, further more preferably 50 or less, further more preferably 40 or less. More specifically, the content ratio is preferably 5 to 80, more preferably 7.5 to 75, even more preferably 10 to 70, further more preferably 20 to 50, further more preferably 20 to 40.

The content ratio by mass of the component (B) to the component (C) in the emulsion cosmetic material of the present invention [(B)/(C)] is, from the viewpoint of improving emulsion stability, preferably 0.1 or more, more preferably 0.3 or more, even more preferably 0.5 or more, further more preferably 0.7 or more, and is, from the viewpoint of improving disintegrability of preparations at the time of application, and from the viewpoint of suppressing the friction feeling at the time of application, preferably 3 or less, more preferably 2 or less, even more preferably 1 or less. More specifically, the content ratio is preferably 0.1 to 3, more preferably 0.3 to 3, even more preferably 0.5 to 2, further more preferably 0.7 to 1.

The oil-in-water emulsion cosmetic material of the present invention may optionally contain any arbitrary component that is used in accordance with the use of cosmetic materials, in addition to the component (A), the component (B) and the component (C), within a range not detracting from the advantageous effects of the present invention. The arbitrary component includes a UV absorbent, an oil, a surfactant, a water-soluble polymer, a neutralizing agent, a pH regulator, a bactericidal agent, an anti-inflammatory agent, a preservative, a colorant, a chelating agent, a whitening agent, an antiperspirant, an antioxidant and a fragrance material, except the component (A), the component (B) and the component (C).

<Component (D): UV absorbent>

The oil-in-water emulsion cosmetic material of the present invention may contain a UV absorbent as a component (D) within a range not detracting from the advantageous effects of the present invention. The UV absorbent includes at least one organic UV absorbent selected from the group consisting of a liquid organic UV absorbent, and a solid organic UV absorbent.

In the present specification, "liquid" indicates a state of flowability in an environment under 1 atmospheric pressure and 25°C, namely, a state under a temperature condition of a melting point or higher (for an amorphous substance not having a melting point, a state under a temperature condition of a fusion point or higher). Also, "solid" indicates a state not having flowability in an environment under 1 atmospheric pressure and 25°C, namely, a state under a temperature condition lower than a melting point (for an amorphous substance not having a melting point, a state under a temperature condition lower than a fusion point).

The liquid organic UV absorbent includes 2-ethylhexyl paramethoxycinnamate, 2-ethoxyethyl paramethoxycinnamate, isopropyl paramethoxycinnamate/diisopropyl cinnamate mixture, methylbis(trimethylsiloxy)silylisopentyl trimethoxycinnamate, paradimethylamino acid amyl benzoate, paradimethylamino acid 2-ethylhexyl benzoate, ethylene glycol salicylate, 2-ethylhexyl salicylate, benzyl salicylate, homomenthyl salicylate, octocrylene, and dimethicodiethylbenzal malonate.

The solid organic UV absorbent includes hexyl 2-(4-diethylamino-2-hydroxybenzoyl)benzoate, 2,4-bis{[4-(2-ethylhexyloxy)-2-hydroxy]phenyl}-6-(4-methoxyphenyl)-1,3,5-triazine, 2,4,6-tris[4-(2-ethylhexyloxycarbonyl)anilino]-1,3,5-triazine, 4-tert-butyl-4'-methoxydibenzoylmethane, and 2-ethylhexyl dimethoxybenzylidenedioxoimidazolidinepropionate.

Examples of commercial products of these UV absorbents include "UVINUL MC80" (2-ethylhexyl paramethoxycinnamate), "UVINUL A PLUS" (hexyl 2-(4-diethylamino-2-hydroxybenzoyl)benzoate), "TINOSORB S" (2,4-bis{[4-(2-ethylhexyloxy)-2-hydroxy]phenyl}-6-(4-methoxyphenyl)-1,3,5-triazine), and "UVINUL T-150" (2,4,6-tris[4(2-ethylhexyloxycarbonyl)anilino]-1,3,5-triazine) (all by BASF Corporation); "Soft Shade DH" (2-ethylhexyl dimethoxybenzylidenedioxoimidazolidinepropionate) (by Ajinomoto Corporation); and "PARSOL 1789" (4-tert-butyl-4'-methoxydibenzoylmethane), and PARSOL SLX (Polysilicane-15 (dimethicodiethylbenzal malonate) (all by DSM Corporation).

The content of the component (D) in the emulsion cosmetic material of the present invention is, from the viewpoint of reducing the load to skin, preferably less than 3% by mass, more preferably less than 2% by mass, even more preferably less than 1% by mass, further more preferably less than 0.5% by mass, further more preferably 0% by mass, that is, the component (D) is not contained.

Even when the content of the component (D) therein falls within the above range, the emulsion cosmetic material of the present invention still has an excellent UV protective effect, is excellent in emulsion stability, has good disintegrability of preparations at the time of application, and can express an effect excellent in the feel in use in that the friction feeling at the time of application and the tensive feeling after application are suppressed. From the viewpoint, the emulsion cosmetic material of the present invention can be applied to organic UV absorbent-free non-chemical sunscreen cosmetic materials that have become attracted attention these days as skin-friendly cosmetics.

<Component (E): Oil except component (D)>

Preferably, the oil-in-water emulsion cosmetic material of the present invention further contain any other oil than the component (D), as a component (E), from the viewpoint of incorporating the component (A) therein to better emulsion stability.

The component (E) is preferably a nonvolatile oil, and includes an ester oil, a silicone oil, a hydrocarbon oil, a higher alcohol, and higher fatty acid.

The nonvolatile oil includes those liquid or solid at 25°C, but preferably contains a liquid one at 25°C. The definition of "liquid" and "solid" is as described above.

"Nonvolatile" in the present specification means that the evaporation amount at 25°C for 6 hours to be measured according to the following method is less than 20%.

Measurement method: A piece of filter paper having a diameter of 90 mm is put in a glass-made laboratory dish having a diameter of 120 mm, 1 g of a sample is put on the filter paper, and stored in a room (25°C) at 65% RH for 6 hours. Before and after the storage, the sample mass is measured, and the evaporation amount is calculated according to the following formula.

$$\text{Evaporation amount (\%)} = [(\text{mass of sample before storage - mass of sample after storage})/(\text{mass of sample before storage})] \times 100$$

Examples of the nonvolatile liquid ester oil include at least one selected from the group consisting of isononyl isononanoate, isotridecyl isononanoate, isopropyl myristate, isocetyl myristate, octyldodecyl myristate, isopropyl palmitate, ethylhexyl palmitate, 2-hexyldecyl palmitate, glyceryl tri-2-ethylhexanoate, di-2-ethylhexyl sebacate, diisopropyl sebacate, glyceryl tri(caprylate/caprate), diisostearyl malate, diethylene glycol dicaprylate, neopentyl glycol dicaprylate, neopentyl glycol di-2-ethylhexanoate, and alkyl benzoates such as alkyl (C12 to 15) benzoates.

Among the above, the nonvolatile liquid ester oil is, from the viewpoint of incorporating the component (A) therein to better emulsion stability, preferably at least one selected from the group consisting of a monoester of a branched fatty acid having 8 or more and 18 or less carbon atoms and a branched alcohol having 2 or more and 22 or less carbon atoms, a triester of a branched fatty acid having 6 or more and 18 or less carbon atoms and glycerin, a diester of a dicarboxylic acid having 2 or more and 18 or less carbon atoms and a branched alcohol having 2 or more and 18 or less carbon atoms, a diester of a fatty acid having 6 or more and 18 or less carbon atoms and a branched alcohol having 2 or more and 10 or less carbon atoms, and an alkyl (C12 to 15) benzoate (e.g., "Finsolv TN (by Innospec Active Chemicals LLC)), more preferably a monoester of a branched fatty acid having 8 or more and 18 or less carbon atoms and a branched alcohol having 2 or more and 22 or less carbon atoms, even more preferably at least one selected from the group consisting of isononyl isononanoate and isotridecyl isononanoate.

The nonvolatile liquid silicone oil is, from the viewpoint of incorporating the component (A) therein to better emulsion stability, preferably dimethylpolysiloxane, more preferably dimethylpolysiloxane having a kinematic viscosity at 25°C of 20 mm$^2$/s or less.

The kinematic viscosity at 25°C of the silicone oil can be measured with an Ubbelohde viscometer according to ASTM D 445-46T or JIS Z 8803.

The nonvolatile liquid hydrocarbon oil includes liquid paraffin, hydrogenated polyisobutene (liquid isoparaffin, heavy liquid isoparaffin), cycloparaffin, liquid ozocerite, squalene, squalane, pristane, $\alpha$-olefin oligomer, polybutene, and isohexadecane.

Examples of the nonvolatile liquid higher alcohol include a higher alcohol having 12 or more and 24 or less carbon atoms, specifically oleyl alcohol, 2-decyltetradecinol, dodecanol, isostearyl alcohol, and 2-octyldodecanol.

Examples of the nonvolatile liquid higher fatty acid include a fatty acid having 12 or more and 22 or less carbon atoms, specifically oleic acid, isostearic acid, linolic acid and linoleic acid.

From the viewpoint of bettering emulsion stability, preferably, the nonvolatile oil to be used as the component (E) in the oil-in-water emulsion cosmetic material of the present invention further contains a nonvolatile solid oil at 25°C.

The nonvolatile solid oil at 25°C includes a solid ester oil such as glyceryl monostearate and glyceryl monomyristate; a solid silicone oil such as alkylmodified silicone; a solid hydrocarbon oil such as vaseline; a solid higher alcohol such as cetyl alcohol, stearyl alcohol, and behenyl alcohol; and a solid higher fatty acid such as lauric acid, myristic acid, palmitic acid, stearic acid, behenic acid, 12-hydroxystearic acid and lanolin fatty acid.

Among these, the component (E) is, from the viewpoint of incorporating the component (A) therein to better emulsion stability, preferably at least one selected from the group consisting of an ester oil, a silicone oil and a higher alcohol.

One alone or two or more kinds of the above-mentioned oils can be used either singly or as combined.

The content of the component (E) in the emulsion cosmetic material of the present invention is, from the viewpoint of incorporating the component (A) therein to better emulsion stability, preferably 5% by mass or more, more preferably 10% by mass or more, even more preferably 15% by mass or more, further more preferably 17% by mass or more, and is preferably 30% by mass or less, more preferably 27% by mass or less, even more preferably 25% by mass or less. More specifically, the content is preferably 5 to 30% by mass, more preferably 10 to 27% by mass, even more preferably 15 to 25% by mass, further more preferably 17 to 25% by mass.

<Component (F): Water-soluble polymer>

The oil-in-water emulsion cosmetic material of the present invention preferably contains, as a component (F), any other water-soluble polymer having a thickening effect than the component (B), from the viewpoint of attaining good emulsion stability.

Not specifically limited, the component (F) may be any one usable in ordinary cosmetic materials, and any of natural polymers, semi-synthetic polymers and synthetic polymers except the component (B) are usable here.

Examples of natural polymers include xanthane gum, carrageenan, and alginic acid. Above all, from the viewpoint of attaining good emulsion stability, xanthane gum is preferred.

Examples of semi-synthetic polymers include modified polysaccharides such as hydroxy cellulose, hydroxypropyl cellulose, sodium carboxymethyl cellulose, methyl cellulose, hydroxymethyl cellulose and cationized cellulose.

Examples of synthetic polymers except the component (B) include acrylic polymers except the component (B) such as carbomer (crosslinked polyacrylic acid), polyacrylic acid, sodium polyacrylate, (acrylic acid/alkyl methacrylate) copolymer, polyacrylamide, and (acrylamide/ammonium acrylate) copolymer; and polyvinyl pyrrolidone, polyvinyl alcohol, and cationized polyvinyl pyrrolidone.

Commercial products of carbomers include "Carbopol 910", "Carbopol 934", "Carbopol 940", "Carbopol 941", "Carbopol 980", and "Carbopol 981" (all by Lubrizol Advanced Materials Corporation).

Commercial products of (acrylic acid/alkyl methacrylate) copolymer include "Carbopol 1382", "Carbopol ETD2020", "PEMULEN TR-1", and "PEMULEN TR-2" (all by Lubrizol Advanced Materials Corporation).

Commercial products of polyacrylamide include "SEPIGEL 305" (by SEPPIC Corporation).

The content of the component (F) in the emulsion cosmetic material of the present invention is, from the viewpoint of attaining good emulsion stability, preferably 0.01% by mass or more, more preferably 0.05% by mass or more, even more preferably 0.1% by mass or more, and is, from the viewpoint of bettering the feeling in use, preferably 5% by mass or less, more preferably 3% by mass or less, even more preferably 1% by mass or less. More specifically, the content is preferably 0.01 to 5% by mass, more preferably 0.05 to 3% by mass, even more preferably 0.1 to 1% by mass.

(Aqueous medium)

The oil-in-water emulsion cosmetic material of the present invention contains at least water as an aqueous medium.

Examples of the aqueous medium except water include a saturated monoalcohol having 1 or more and 3 or less carbon atoms, such as ethanol, and isopropanol; and a polyalcohol such as ethylene glycol, diethylene glycol, triethylene glycol, polyethylene glycol (having an average molecular weight of less than 650), propylene glycol, dipropylene glycol, polypropylene glycol (having an average molecular weight of less than 650), isoprene glycol, 1,3-butylene glycol, glycerin, diglycerin, and polyglycerin. Among these, preferred is a polyalcohol, more preferred is at least one selected from the group consisting of ethylene glycol, diethylene glycol, propylene glycol, dipropylene glycol, 1,3-butylene glycol and glycerin, and even more preferred is at least one selected from the group consisting of dipropylene glycol, 1,3-butylene glycol and glycerin. One alone or two or more kinds of these alcohols can be used either singly or as combined.

The content of the aqueous medium in the emulsion cosmetic material of the present invention may fall within a range capable of providing an oil-in-water cosmetic material, and is, from the viewpoint of improving emulsion stability, preferably 40% by mass or more, more preferably 45% by mass or more, even more preferably 47% by mass or more, and is preferably 90% by mass or less, more preferably 80% by mass or less, even more preferably 75% by mass or less. More specifically, the content is preferably 40 to 90% by mass, more preferably 45 to 80% by mass, even more preferably 47 to 75% by mass.

In the case where the emulsion cosmetic material of the present invention contains a polyalcohol, the content of the polyalcohol in the emulsion cosmetic material is, from the viewpoint of improving emulsion stability, preferably 0.5% by mass or more, more preferably 1% by mass or more, even more preferably 2% by mass or more, and is preferably 7% by mass or less, more preferably 5% by mass or less, even more preferably 4% by mass or less. More specifically, the content is preferably 0.5 to 7% by mass, more preferably 1 to 5% by mass, even more preferably 2 to 4% by mass.

The emulsion cosmetic material of the present invention can be favorably used as a hair cosmetic material such as shampoo, rinse or conditioner; and a skin cosmetic material such as face wash, cleansing cosmetic material, sunscreen cosmetic material, facial pack or massage cosmetic material. Among these, as having an excellent UV protective effect, the emulsion cosmetic material of the present invention is favorably applied to a sunscreen cosmetic material (lotion, cream, emulsion, beauty essence), a tanning material, a makeup base cosmetic material and a foundation, for use for sunscreen.

Regarding the preparation form thereof, the emulsion cosmetic material of the present invention is applicable to a liquid, an emulsion, a cream, a paste, a solid and a multilayer, and is further applicable to a sheet, a spray or a mousse.

(Production method for oil-in-water emulsion cosmetic material)

The production method for the oil-in-water emulsion cosmetic material of the present invention is not specifically limited, and any known method can be appropriately employed in accordance with the preparation form of the oil-in-water emulsion cosmetic material. For example, an example of the method includes a step of blending the component (A), the component (B), the component (C) and, as needed, the above-mentioned optional components, and uniformly mixing them with a homomixer or the like.

As the production method for the emulsion cosmetic material of the present invention, above all, from the viewpoint of increasing the dispersibility of the component (A) in the emulsion cosmetic material to improve the UV protective effect, emulsion stability and disintegrability of preparations at the time of application and also from the viewpoint of suppressing the friction feeling at the time of application and the tensive feeling after application, preferred is a method including a step of mixing and emulsifying a dispersion prepared by dispersing the component (A) in an oily phase component containing the component (C) and, as needed, the above-mentioned optional oil-soluble components, and a preparation product of an aqueous phase component containing the component (B), an aqueous medium and, as needed, the above-mentioned optional water-soluble components, and more preferred is a method including the following step I to step III.

Step I: a step of adding the component (A) to the component (C) and, as needed, the above-mentioned oil-soluble oily phase component, and uniformly mixing them to give a dispersion (i) where the component (A) has been dispersed,

Step II: a step of uniformly mixing an aqueous phase component containing the component (B), an aqueous medium and, as needed, the above-mentioned optional water-soluble components to give a preparation product (ii),

Step III: a step of adding the dispersion (i) prepared in the step I to the preparation product (ii) prepared in the step II, and uniformly mixing and emulsifying them to give an oil-in-water emulsion cosmetic material.

The step I preferably includes the following step I-1 and step I-2, from the viewpoint of increasing the dispersibility of the component (A) in the emulsion cosmetic material.

Step I-1: a step of uniformly mixing an oily phase component containing the component (C) and, as needed, the above-mentioned optional oil-soluble component to prepare a preparation product (i'),

Step I-2: a step of adding the component (A) to the preparation product (I') prepared in the step I-1 and uniformly mixing them to prepare a dispersion (i) where the component (A) has been dispersed.

Preferably, mixing the oily phase component in the step I-1 and mixing the aqueous phase component in the step II each are carried out with heating and stirring in a temperature range of preferably 40°C or higher and 90°C or lower.

Preferably, in the step II, the mixture is, after once cooled to a temperature range of 15°C or higher and 35°C or lower, further uniformly mixed to give the preparation product (ii).

In the step III, also preferably, while the preparation product (ii) prepared in the step II is stirred, the dispersion (i) prepared in the step I is kept at a temperature of preferably 40°C or higher and 90°C or lower and added thereto, and unfirmly mixed and emulsified.

Regarding the above-mentioned embodiments, the present invention further discloses the following embodiments.

<1> An oil-in-water emulsion cosmetic material containing the following component (A), component (B) and component (C):

Component (A): at least one selected from the group consisting of hydrophobized fine-particulate titanium oxide and hydrophobized fine-particulate zinc oxide having an average primary particle diameter of 1 nm or more and 70 nm or less,
Component (B): a copolymer containing a structural unit derived from 2-acrylamido-2-methylpropanesulfonic acid and a structural unit derived from an acrylic monomer,
Component (C): a betaine-type surfactant, wherein:
the content of the component (A) is 9% by mass or more and 27% by mass or less.

<2> An oil-in-water emulsion cosmetic material containing the following component (A), component (B) and component (C):

Component (A): at least one selected from the group consisting of hydrophobized fine-particulate titanium oxide and hydrophobized fine-particulate zinc oxide having an average primary particle diameter of 5 nm or more and 50 nm or less,
Component (B): a copolymer containing a structural unit derived from 2-acrylamido-2-methylpropanesulfonic acid and a structural unit derived from an acrylic monomer,
Component (C): a betaine-type surfactant, wherein:
the content of the component (A) is 13% by mass or more and 20% by mass or less.

<3> The oil-in-water emulsion cosmetic material according to the above <1> or <2>, wherein the acrylic monomer is at least one selected from the group consisting of acrylic acid, acrylamide, dimethylacrylamide, alkyl acrylate, alkyl methacrylate, hydroxyalkyl acrylate and polyoxyethylene alkyl methacrylate (average addition molar number of ethylene oxide: 4 or more and 30 or less, preferably 10 or more and 30 or less).
<4> The oil-in-water emulsion cosmetic material according to any of the above <1> to <3>, wherein the component (B) is at least one selected from the group consisting of (Na acryloyldimethyltaurate/Na acrylate) copolymer, (ammonium acryloyldimethyltaurate/beheneth-25 methacrylate) crosspolymer, and (ammonium acryloyldimethyltaurate/dimethylacrylamide/lauryl methacrylate/laureth-4 methacrylate) crosspolymer.
<5> The oil-in-water emulsion cosmetic material according to any of the above <1> to <4>, wherein the component (B) is (Na acryloyldimethyltaurate/Na acrylate) copolymer.

<6> The oil-in-water emulsion cosmetic material according to any of the above <1> to <5>, wherein the component (C) is hydrogenated lecithin.

<7> The oil-in-water emulsion cosmetic material according to any of the above <1> to <6>, wherein the content of UV absorbent is less than 3% by mass.

<8> The oil-in-water emulsion cosmetic material according to any of the above <1> to <6>, wherein the content of UV absorbent is less than 1% by mass.

<9> The oil-in-water emulsion cosmetic material according to any of the above <1> to <8>, wherein the content of the component (B) is 0.1% by mass or more and 3% by mass or less.

<10> The oil-in-water emulsion cosmetic material according to any of the above <1> to <8>, wherein the content of the component (B) is 0.25% by mass or more and 0.5% by mass or less.

<11> The oil-in-water emulsion cosmetic material according to any of the above <1> to <10>, wherein the content of the component (C) is 0.1% by mass or more and 3% by mass or less.

<12> The oil-in-water emulsion cosmetic material according to any of the above <1> to <10>, wherein the content of the component (C) is 0.3% by mass or more and 0.7% by mass or less.

<13> The oil-in-water emulsion cosmetic material according to any of the above <1> to <12>, wherein the content ratio by mass of the component (A) to the component (C) [(A)/(C)] is 7.5 or more and 75 or less.

<14> The oil-in-water emulsion cosmetic material according to any of the above <1> to <12>, wherein the content ratio by mass of the component (A) to the component (C) [(A)/(C)] is 20 or more and 40 or less.

<15> The oil-in-water emulsion cosmetic material according to any of the above <1> to <14>, wherein the content ratio by mass of the component (A) to the component (B) [(A)/(B)] is 10 or more and 90 or less.

<16> The oil-in-water emulsion cosmetic material according to any of the above <1> to <14>, wherein the content ratio by mass of the component (A) to the component (B) [(A)/(B)] is 30 or more and 50 or less.

<17> The oil-in-water emulsion cosmetic material according to any of the above <1> to <16>, wherein the content ratio by mass of the component (B) to the component (C) [(B)/(C)] is 0.3 or more and 3 or less.

<18> The oil-in-water emulsion cosmetic material according to any of the above <1> to <16>, wherein the content ratio by mass of the component (B) to the component (C) [(B)/(C)] is 0.7 or more and 1 or less.

<19> The oil-in-water emulsion cosmetic material according to any of the above <1> to <18>, wherein the component (A) contains a hydrophobized fine-particulate zinc oxide and the content of the hydrophobized fine-particulate zinc oxide in the component (A) is 60% by mass or more.

<20> The oil-in-water emulsion cosmetic material according to any of the above <1> to <18>, wherein the component (A) contains a hydrophobized fine-particulate titanium oxide and the content of the hydrophobized fine-particulate titanium oxide in the component (A) is 50% by mass or more.

<21> The oil-in-water emulsion cosmetic material according to any of the above <1> to <20>, wherein the hydrophobization for the component (A) is at least one selected from the group consisting of silicone treatment, alkylalkoxysilane treatment and fatty acid treatment.

<22> The oil-in-water emulsion cosmetic material according to any of the above <1> to <21>, which is for use for sunscreen.

<23> An oil-in-water emulsion cosmetic material containing the following component (A), component (B) and component (C):

Component (A): at least one selected from the group consisting of a hydrophobized fine-particulate titanium oxide and a hydrophobized fine-particulate zinc oxide, having an average primary particle diameter of 1 nm or more and 70 nm or less.

Component (B): at least one selected from the group consisting of (Na acryloyldimethyltaurate/Na acrylate) copolymer, (ammonium acryloyldimethyltaurate/beheneth-25 methacrylate) crosspolymer, and (ammonium acryloyldimethyltaurate/dimethylacrylamide/lauryl methacrylate/laureth-4 methacrylate) crosspolymer,

Component (C): hydrogenated lecithin, wherein:

the content of the component (A) is 9% by mass or more and 27% by mass or less,
the content of UV absorbent is less than 3% by mass, and
which is for use for sunscreen.

<24> An oil-in-water emulsion cosmetic material containing the following component (A), component (B) and component (C):

Component (A): at least one selected from the group consisting of a hydrophobized fine-particulate titanium oxide and a hydrophobized fine-particulate zinc oxide, having an average primary particle diameter of 5 nm or more and 50 nm or less.

Component (B): at least one selected from the group consisting of (Na acryloyldimethyltaurate/Na acrylate) copolymer, (ammonium acryloyldimethyltauratelbeheneth-25 methacrylate) crosspolymer, and (ammonium acryloyldimethyltaurate/dimethylacrylamide/lauryl methacrylate/laureth-4 methacrylate) crosspolymer,
Component (C): hydrogenated lecithin, wherein:

the content of the component (A) is 13% by mass or more and 20% by mass or less,
the content of UV absorbent is less than 1% by mass, and
which is for use for sunscreen.

<25> The oil-in-water emulsion cosmetic material according to the above <23> or <24>, wherein the content ratio by mass of the component (A) to the component (C) [(A)/(C)] is 7.5 or more and 75 or less.
<26> The oil-in-water emulsion cosmetic material according to the above <23> or <24>, wherein the content ratio by mass of the component (A) to the component (C) [(A)/(C)] is 20 or more and 40 or less.
<27> The oil-in-water emulsion cosmetic material according to any of the above <23> to <26>, wherein the content ratio by mass of the component (A) to the component (B) [(A)/(B)] is 10 or more and 90 or less.
<28> The oil-in-water emulsion cosmetic material according to any of the above <23> to <26>, wherein the content ratio by mass of the component (A) to the component (B) [(A)/(B)] is 30 or more and 50 or less.
<29> The oil-in-water emulsion cosmetic material according to any of the above <23> to <28>, wherein the content ratio by mass of the component (B) to the component (C) [(B)/(C)] is 0.3 or more and 3 or less.
<30> The oil-in-water emulsion cosmetic material according to any of the above <23> to <28>, wherein the content ratio by mass of the component (B) to the component (C) [(B)/(C)] is 0.7 or more and 1 or less.

Examples

Hereinunder the present invention is described in detail with reference to Examples and Comparative Examples, to which, however, the present invention is not limited.

(Average primary particle diameter of component (A))

The average primary particle diameter of the component (A) was measured according to the following method.
For a measurement sample having a shape except tabular shapes, a dispersion of the measurement sample that had been prepared previously was put on a transmission electron microscope (TEM) (trade name "JEM 1400 Plus", by JEOL Ltd.), dried thereon in air, and then observed under the condition of an observation magnification of 50,000 times. A maximum minor diameter of each of 300 primary particles on the image was measured, and a number-average value thereof was referred to as an average primary particle diameter. Here, the maximum minor diameter means a minor diameter having a maximum length of the minor diameter that crosses a major diameter at right angles.
For a measurement sample having a tabular shape, a thickness of each of 300 primary particles was measured in the image taken according to the above-mentioned method and under the condition of observation magnification also mentioned above, and a number-average value thereof was referred to as an average primary particle diameter.
A dispersion of the measurement sample was prepared by adding 95 g of a solvent, ethanol to 5 g of the measurement sample followed by ultrasonic dispersion thereof.

Examples 1 to 10, Comparative Examples 1 to 5

According to the formulation shown in Table 1, oil-in-water emulsion cosmetic materials were produced according to the following method.
The components 9 to 14 shown in Table 1 were uniformly mixed, and melted by heating at 80°C to prepare a preparation product (i') (step I-1). Next, while the resultant preparation product (i') was kept at 80°C, the components 1 to 4 shown in Table 1 were added, uniformly mixed and dispersed to prepare a dispersion (i) (step I-2).
Separately, the components 5 to 8 and 15 to 18 shown in Table 1 were melted by heating at 40°C, then spontaneously cooled down to 25°C, and uniformly mixed to prepare a preparation product (ii) (step II).
While the resultant preparation product (ii) was stirred, the dispersion (i) kept at 80°C was added thereto, uniformly mixed and emulsified with a homomixer to give an oil-in-water emulsion cosmetic material (step III).
The resultant oil-in-water emulsion cosmetic material was evaluated for (1) UV protection performance, (2) emulsion stability, and (3) feeling in use (disintegrability of preparation at the time of application, absence of friction feeling at the time of application, and absence of tensive feeling after application). The results are shown in Table 1.

(1) UV protection performance

The oil-in-water emulsion cosmetic material shown in Table 1 was uniformly applied onto a square PMMA plate ("HD6" by HelioScreen Corporation) in a coating amount of 1.3 mg/cm$^2$, and dried for 15 minutes to give a measurement sample. Similarly, glycerin was applied onto a PMMA plate and dried for 15 minutes to give a control sample. Using an SPF analyzer "UV-2000S" (by Labsphere Corporation), an in-vitro SPF value of each sample was calculated from the measurement results of the absorption spectrum thereof (measurement wavelength 290 to 450 nm), and an average in-vitro SPF value at 9 spots of each sample was referred to as a UV protection performance (SPF value).

(2) Emulsion stability

Immediately after preparation and after storage at 40°C for 1 month, the outward appearance of oil-in-water emulsion cosmetic material shown in Table 1 was visually observed, and evaluated according to the following acceptance criteria.

[Acceptance criteria]

A: Neither separation nor gelation was observed immediately after cosmetic preparation, and even after storage at 40°C for 1 month, neither separation nor gelation was also observed.
B: Neither separation nor gelation was observed immediately after cosmetic preparation, and after storage at 40°C for 1 month, slight separation or gelation was observed.
C: Though neither separation nor gelation was observed immediately after cosmetic preparation, after storage at 40°C for 1 month, obvious separation or gelation was observed.
D: Immediately after cosmetic preparation, separation or gelation was observed.

(3) Feeling in use (disintegrability of preparation at the time of application, absence of friction feeling at the time of application, and absence of tensive feeling after application)

10 expert panelists tried the oil-in-water emulsion cosmetic materials shown in Table 1. 0.02 mL of each oil-in-water emulsion cosmetic material was applied to the inside of forearm to draw a circle having a diameter of 3 cm thereon at 25°C and 57% RH, spread thereon taking one minute, and evaluated for the feeling in use in point of "disintegrability of preparation at the time of application", "absence of friction feeling at the time of application", and "absence of tensive feeling after application", according to the following evaluation criteria. Based on the average value given by the 10 panelists, the cosmetic materials were evaluated based on the following acceptance criteria.
In addition, after the cosmetic material was spread thereon, the inside of forearm was visually checked for transparency and, as a result, the transparency was higher in Examples 1 to 3 than in Example 4.

[Evaluation criteria]

5: Extremely good.
4: Good.
3: Average.
2: Relatively bad.
1: Bad.

[Acceptance criteria]

A: 4.0 or more and 5.0 or less.
B: 3.0 or more and less than 4.0.
C: 2.0 or more and less than 3.0.
D: 1.0 or more and less than 2.0.

Table 1

| | Components | | | Example | | | | | | | | | | Comparative Example | | | | |
|---|---|---|---|---|---|---|---|---|---|---|---|---|---|---|---|---|---|---|---|
| | No. | Kind | 1 | 2 | 3 | 4 | 5 | 6 | 7 | 8 | 9 | 10 | 1 | 2 | 3 | 4 | 5 |
| Formulation of Oil-in-Water Emulsion Cosmetic Material (mass%) | 1 (A) | dimethylpolysiloxane/n-octylsilylated low-temperature fired zinc oxide average primary particle diameter 30 nm*1 | 15 | 10 | 25 | | 15 | 15 | 15 | 15 | 15 | 15 | 5 | | | 15 | 15 |
| | 2 | n-octylsilylated hydrous silicate-treated fine-particulate titanium oxide average primary particle diameter 10 nm*2 | | | | 15 | | | | | | | | | | | |
| | 3 (A') | ASI-aluminum hydroxide/hydrous silicate/zinc oxide-coated titanium oxide average primary particle diameter 270 nm*3 | | | | | | | | | | | | | 15 | | |
| | 4 | hydrous silicate-treated fine-particulate titanium oxide average primary particle diameter 10 nm*4 | | | | | | | | | | | | | | 15 | |
| | 5 (B) | (Na acryloyldimethyltaurate/Na acrylate) copolymer/isohexadecane/Polysorbate 80*5 | 1 | 1 | 1 | 1 | 0.5 | 3 | | | 1 | 1 | 1 | 1 | 1 | | 1 |
| | 6 | (ammonium acryloyldimethyltaurate/ beheneth-25 methacrylate) crosspolymer*6 | | | | | | | 0.35 | | | | | | | | |
| | 7 | (ammonium acryloyldimethyltaurate/ dimethylacrylamide/ lauryl methacrylate/ laureth-4 methacrylate) crosspolymer*7 | | | | | | | | 0.35 | | | | | | | |
| | 8 (B') | carboxyvinyl polymer*8 | | | | | | | | | | | | | | | 1 |
| | 9 (C) | hydrogenated soybean lecithin*9 | 0.5 | 0.5 | 0.5 | 0.5 | 0.5 | 0.5 | 0.5 | 0.5 | 0.2 | 2 | 0.5 | 0.5 | 0.5 | 0.5 | |
| | 10 (C') | polyoxyethylene hydrogenated castor oil*10 | | | | | | | | | | | | | | | 0.5 |
| | 11 | isotridecyl isononanoate | 10 | 10 | 10 | 10 | 10 | 10 | 10 | 10 | 10 | 10 | 10 | 10 | 10 | 10 | 10 |
| | 12 | isononyl isononanoate | 5 | 5 | 5 | 5 | 5 | 5 | 5 | 5 | 5 | 5 | 5 | 5 | 5 | 5 | 5 |
| | 13 | dimethylpolysiloxane kinematic viscosity 6 mm²/s*11 | 5 | 5 | 5 | 5 | 5 | 5 | 5 | 5 | 5 | 5 | 5 | 5 | 5 | 5 | 5 |
| | 14 | behenyl alcohol | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | 15 | xanthane gum | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| | 16 | 1,3-butylene glycol | 3 | 3 | 3 | 3 | 3 | 3 | 3 | 3 | 3 | 3 | 3 | 3 | 3 | 3 | 3 |
| | 17 | disodium edetate | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 |
| | 18 | pure water | balance | balance | balance | balance | balance | balance | balance | balance | balance | balance | balance | balance | balance | balance | balance |
| | | total | 100 | 100 | 100 | 100 | 100 | 100 | 100 | 100 | 100 | 100 | 100 | 100 | 100 | 100 | 100 |
| | | Content (mass%) of component (B) in oil-in-water emulsion cosmetic material | 0.375 | 0.375 | 0.375 | 0.375 | 0.188 | 1.125 | 0.350 | 0.350 | 0.375 | 0.375 | 0.375 | 0.375 | 0.375 | (1) | 0.375 |
| | | Content mass ratio [(A)/(B)] | 40.0 | 26.7 | 66.7 | 40.0 | 80.0 | 13.3 | 42.9 | 42.9 | 40.0 | 40.0 | 13.3 | (40.0) | (40.0) | (15.0) | 40.0 |
| | | Content mass ratio [(A)/(C)] | 30.0 | 20.0 | 50.0 | 30.0 | 30.0 | 30.0 | 30.0 | 30.0 | 75.0 | 7.5 | 10.0 | (30.0) | (30.0) | 30.0 | (30.0) |
| | | Content mass ratio [(B)/(C)] | 0.75 | 0.75 | 0.75 | 0.75 | 0.38 | 2.25 | 0.70 | 0.70 | 1.88 | 0.19 | 0.75 | 0.75 | 0.75 | (2.00) | (0.75) |
| Evaluation | | UV protection performance (SPF value) | 30 | 24 | 43 | 40 | 28 | 35 | 29 | 31 | 27 | 31 | 11 | 8 | 14 | 16 | 15 |
| | | Emulsion stability | A | A | B | A | B | A | A | B | B | A | A | D | D | D | D |
| | | Disintegrability of preparation at the time of application | A | A | B | A | A | B | A | A | A | B | A | B | C | A | A |
| | | Absence of friction feeling at the time of application | A | A | B | A | A | B | A | A | A | B | A | B | C | A | A |
| | | Absence of tensive feeling after application | A | A | B | A | A | A | A | A | A | B | A | B | C | A | A |

Details of the components used in Table 1 are shown below.

*1: "MZY-303M20" by Tayca Corporation (hydrophobized fine-particulate zinc oxide, average primary particle diameter 30 nm)

*2: "STR-100W-OTS" by Sakai Chemical Industry Co., Ltd. (hydrophobized fine-particulate titanium oxide, average primary particle diameter 10 nm)

*3: "BCASI TIO2 MP-701" by Daito Kasei Kogyo Co., Ltd. (hydrophobized pigment-grade titanium oxide, average primary particle diameter 270 nm)

*4: "STR-100W(G)" by Sakai Chemical Industry Co., Ltd. (hydrophilized fine-particulate titanium oxide, average primary particle diameter 10 nm)

*5: "SIMULGEL EG" by SEPPIC Corporation ((Na acryloyldimethyltaurate/Na acrylate) copolymer, isohexadecane, Polysorbate 80) ((Na acryloyldimethyltaurate/Na acrylate) copolymer, active ingredient 37.5% by mass)

*6: "Aristoflex HMB" by Clariant Corporation ((ammonium acryloyldimethyltaurate/beheneth-25 methacrylate)crosspolymer, active ingredient 100% by mass)

*7: "SEPIMAX ZEN" by SEPPIC Corporation ((ammonium acryloyldimethyltaurate/dimethylacrylamide/lauryl methacrylate/laureth-4 methacrylate) crosspolymer, active ingredient 100% by mass)

*8: "Synthalen K" by 3V Sigma Corporation

*9: "PhosphoLipid PCSH70" by Nippon Fine Chemical Co., Ltd. (phosphatidylcholine content: about 70% by mass)

*10: "NIKKOL HCO-60" by Nikko Chemicals Co., Ltd. (nonionic surfactant)

*11: "KF-96A-6CS" by Shin-Etsu Chemical Industry Co., Ltd.

From Table 1, it is known that the oil-in-water emulsion cosmetic materials of Examples of the present invention have an excellent UV protective effect, are excellent in emulsion stability, are disintegrable as preparations at the time of application, and are suppressed in the friction feeling at the time of application and in the tensive feeling after application, as compared with the oil-in-water emulsion cosmetic materials of Comparative Examples.

In Comparative Example 1, the content of the component (A) in the oil-in-water emulsion cosmetic material is less than 7% by mass, and therefore the UV protective effect is low.

In Comparative Example 2, a hydrophobized pigment-grade titanium oxide (average primary particle diameter 270 nm) is used as a metal oxide powder, and therefore the UV protective effect is low and emulsion stability is insufficient.

In Comparative Example 3, a hydrophilized fine-particulate titanium oxide is used as a metal oxide powder, and therefore the UV protective effect is low, and the feeling in use (disintegrability of preparation at the time of application, absence of friction feeling at the time of application, absence of tensive feeling after application) is poor.

In Comparative Example 4 and Comparative Example 5, the oil-in-water emulsion cosmetic material does not contain an aqueous phase thickener containing a structure derived from 2-acrylamido-2-methylpropanesulfonic acid or an amphoteric surfactant, and therefore the UV protective effect is low and emulsion stability is insufficient.

**Claims**

1.   An oil-in-water emulsion cosmetic material comprising the following component (A), component (B) and component (C):

Component (A): a hydrophobized fine-particulate metal oxide having an average primary particle diameter of less than 100 nm,
Component (B): an aqueous phase thickener containing a structure derived from 2-acrylamido-2-methylpropanesulfonic acid,
Component (C): an amphoteric surfactant, wherein:
the content of the component (A) is 7% by mass or more and 30% by mass or less.

2.   The oil-in-water emulsion cosmetic material according to claim 1, wherein the component (B) is at least one selected from the group consisting of (Na acryloyldimethyltaurate/Na acrylate) copolymer, (ammonium acryloyldimethyltaurate/beheneth-25 methacrylate) crosspolymer, and (ammonium acryloyldimethyltaurate/dimethylacrylamide/lauryl methacrylate/laureth-4 methacrylate) crosspolymer.

3.   The oil-in-water emulsion cosmetic material according to claim 1 or 2, wherein the component (B) is a (Na acryloyldimethyltaurate/Na acrylate) copolymer.

4.   The oil-in-water emulsion cosmetic material according to any of claims 1 to 3, wherein the component (C) is a hydrogenated lecithin.

5. The oil-in-water emulsion cosmetic material according to any of claims 1 to 4, wherein the content of UV absorbent is less than 3% by mass.

6. The oil-in-water emulsion cosmetic material according to any of claims 1 to 5, wherein the content ratio by mass of the component (A) to the component (C) [(A)/(C)] is 7.5 or more and 75 or less.

7. The oil-in-water emulsion cosmetic material according to any of claims 1 to 6, wherein the content ratio by mass of the component (A) to the component (B) [(A)/(B)] is 10 or more and 90 or less.

8. The oil-in-water emulsion cosmetic material according to any of claims 1 to 7, wherein the metal oxide of the component (A) is at least one selected from the group consisting of titanium oxide and zinc oxide.

9. The oil-in-water emulsion cosmetic material according to any of claims 1 to 8, wherein hydrophobization treatment for the component (A) is at least one selected from the group consisting of silicone treatment, alkylalkoxysilane treatment and fatty acid treatment.

10. The oil-in-water emulsion cosmetic material according to any of claims 1 to 9, which is for use for sunscreen.

## INTERNATIONAL SEARCH REPORT

| International application No. |
| --- |
| PCT/JP2021/007532 |

### A. CLASSIFICATION OF SUBJECT MATTER
Int.Cl. A61Q17/04(2006.01)i, A61K8/06(2006.01)i, A61K8/27(2006.01)i,
A61K8/29(2006.01)i, A61K8/55(2006.01)i, A61K8/81(2006.01)i
FI: A61K8/29, A61K8/06, A61K8/27, A61K8/81, A61K8/55, A61Q17/04

According to International Patent Classification (IPC) or to both national classification and IPC

### B. FIELDS SEARCHED

Minimum documentation searched (classification system followed by classification symbols)
Int.Cl. A61Q1/00-90/00, A61K8/00-8/99

Documentation searched other than minimum documentation to the extent that such documents are included in the fields searched
Published examined utility model applications of Japan      1922-1996
Published unexamined utility model applications of Japan    1971-2021
Registered utility model specifications of Japan            1996-2021
Published registered utility model applications of Japan    1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2017-088599 A (KOSE CORPORATION) 25 May 2017 (2017-05-25), paragraphs [0027], [0062] | 1-10 |
| X Y | WO 2014/185315 A1 (FUJIFILM CORPORATION) 20 November 2014 (2014-11-20), paragraphs [0015], [0017], [0065], [0094], [0095], [0110] | 1-3, 5-10 4 |
| X Y | JP 2019-094280 A (KAO CORPORATION) 20 June 2019 (2019-06-20), paragraphs [0020]-[0022], [0077], [0078], [0082] | 1-3, 5-10 4 |
| Y | JP 2018-108994 A (KAO CORPORATION) 12 July 2018 (2018-07-12), paragraphs [0016]-[0020] | 4 |

☒  Further documents are listed in the continuation of Box C.      ☒  See patent family annex.

| * | Special categories of cited documents: | | "T" | later document published after the international filing date or priority date and not in conflict with the application but cited to understand the principle or theory underlying the invention |
| --- | --- | --- | --- | --- |
| "A" | document defining the general state of the art which is not considered to be of particular relevance | | | |
| "E" | earlier application or patent but published on or after the international filing date | | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | | |
| "P" | document published prior to the international filing date but later than the priority date claimed | | "&" | document member of the same patent family |

| Date of the actual completion of the international search | Date of mailing of the international search report |
| --- | --- |
| 15 April 2021 | 27 April 2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
|---|
| PCT/JP2021/007532 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2014-118386 A (TOKIWA CORP.) 30 June 2014 (2014-06-30) | 1-10 |
| A | JP 2019-172634 A (NARIS COSMETICS CO., LTD.) 10 October 2019 (2019-10-10) | 1-10 |

Form PCT/ISA/210 (second sheet) (January 2015)

| INTERNATIONAL SEARCH REPORT | International application No. |
|---|---|
| Information on patent family members | PCT/JP2021/007532 |

| | | | |
|---|---|---|---|
| JP 2017-088599 A | 25 May 2017 | (Family: none) | |
| WO 2014/185315 A1 | 20 November 2014 | EP 2997955 A1<br>paragraphs [0027], [0030], [0096],<br>[0097], [0142], [0147]<br>CN 105188645 A<br>KR 10-2015-0139963 A | |
| JP 2019-094280 A | 20 June 2019 | (Family: none) | |
| JP 2018-108994 A | 12 July 2018 | CN 110114053 A<br>KR 10-2019-0101359 A | |
| JP 2014-118386 A | 30 June 2014 | CN 103860396 A<br>KR 10-2014-0078572 A | |
| JP 2019-172634 A | 10 October 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018108994 A
- JP 2019094280 A

- JP 2001186898 A